# EUROPEAN PATENT SPECIFICATION

(11) **EP 2 659 916 B1**
(45) Date of publication and mention of the grant of the patent: **16.11.2016**
(21) Application number: 11853300.9
(22) Date of filing: 21.12.2011
(51) Int. Cl.: A61M 1/02

(54) **BLOOD PROCESSING FILTER, BLOOD CIRCUIT SYSTEM, AND CENTRIFUGATION METHOD**
BLUTVERARBEITUNGSFILTER, BLUTKREISLAUFSYSTEM UND ZENTRIFUGIERUNGSVERFAHREN
FILTRE DE FRACTIONNEMENT DU SANG, SYSTÈME DE CIRCUITS POUR LE SANG, ET PROCÉDÉ DE CENTRIFUGATION

(30) Priority: 27.12.2010 US 201061427297 P
(43) Date of publication of application: 06.11.2013
(73) Proprietor: Asahi Kasei Medical Co., Ltd., Chiyoda-ku Tokyo 101-8101 (JP)
(72) Inventor: ANDOU, Michiyo, Tokyo 101-8101 (JP)
(74) Representative: dompatent von Kreisler Selting Werner - Partnerschaft von Patent- und Rechtsanwälten mbB
(86) International application number: PCT/JP2011/079712
(87) International publication number: WO 2012/090835

(56) References cited:
- JP-A- 6 245 998
- JP-A- 7 267 871
- JP-A- 2002 541 941
- JP-A- 2003 245 342
- JP-A- 2003 245 342
- JP-A- 2004 537 330
- JP-A- 2006 043 297
- JP-U- 63 166 247
- US-A- 5 092 996
- US-A- 5 527 472
- US-A- 5 626 749
- US-A1- 2001 037 978
- US-A1- 2002 046 967
- US-A1- 2002 113 003
- US-A1- 2008 110 829
- US-B2- 6 629 918

## Description

### Technical Field

The present invention relates to a blood processing filter for removing undesirable components such as aggregates and leukocytes from blood, to a blood circuit system that uses the blood processing filter, and to a centrifugation method that centrifuges blood that is contained in the system.

### Background Art

In the field of blood transfusion, so-called "leukocyte-free blood transfusion" in which a blood preparation is transfused after removing leukocytes contained in the blood preparation is in widespread use. This is because it has been found that relatively minor side effects accompanying blood transfusion, such as headache, nausea, chill, or non hemolytic febrile transfusion reaction, or serious side effects which seriously affect the blood recipient, such as alloantigen sensitization, viral infection, or post-transfusion graft-versus-host disease (GVHD), are mainly caused by leukocytes contained in the blood preparation used for blood transfusion.

Several leukocyte removal methods are available. Of these, a filter method is currently in widespread use due to advantages such as excellent leukocyte removal capability, easy operation, and a low cost. For example, Japanese Patent Laid-Open No. 2000-342680 discloses a leukocyte removal device in which a housing that houses a filter media is composed of a flexible resin.

In this connection, in order to separate blood into a plurality of components to manufacture blood preparations, centrifugation is performed in a state in which the blood is contained in a reservoir bag. In some cases, a separation process is performed in which not only the reservoir bag, but also a blood circuit system in which a filter for filtering a specific component after centrifugation or a recovery bag for recovering a specific component is previously connected to the reservoir bag is inserted into a centrifuge cup of a centrifugal machine.

Another relevant prior art blood processing filter is disclosed in document US2001/0037978 A1.

### Citation List

### Patent Literature

Patent Document 1: Japanese Patent Laid-Open No. 2000-342680

### Summary of Invention

### Technical Problem

In general, many of the aforementioned centrifuge cups have a round shape such as a circular shape in a planar view or an elliptical shape in a planar view. Hence, an operation performed to store a tabular blood processing filter that is difficult to bend inside a centrifuge cup together with a recovery bag or a reservoir bag has been complicated. Further, it is also necessary for the width of the blood processing filter to be less than the width of the centrifuge cup. Hence, in order to broaden the range of adaptation of a blood processing filter with respect to small centrifuge cups, it is preferable to reduce the width of the blood processing filter. However, since it is also necessary to retain a filtration cross-sectional area that is required by a blood processing filter, there is a limit to the extent to which the width of a blood processing filter can be reduced.

An object of the present invention is to provide a blood processing filter, a blood circuit system, and a method for centrifugation that facilitate an operation to store the blood processing filter or blood circuit system in a centrifuge cup, and that easily adapt to small centrifuge cups also, while retaining the filtration cross-sectional area of the blood processing filter.

### Solution to Problem

The present invention provides a blood processing filter such as defined in claim 1.

A case will now be considered in which, for example, the above described blood processing filter is used as one component of a blood circuit system. In this case, when centrifuging blood that is inside a reservoir bag of the blood circuit system, the blood processing filter is stored in the centrifuge cup together with the reservoir bag and the like. Generally, many centrifuge cups of this kind have a round shape such as a circular shape in a planar view or an elliptical shape in a planar view, and many of such centrifuge cups have a round inner surface such as a cylindrical surface or an elliptic cylindrical surface. Since the above described blood processing filter has a curved shape, the blood processing filter can be smoothly inserted into a centrifuge cup by aligning the curved shape of the blood processing filter with the curved shape of an inner wall surface of the centrifuge cup. Hence, an operation to store the blood processing filter in the centrifuge cup is performed with ease. Further, since the blood processing filter has a curved shape, the width thereof can be reduced while retaining the area of the filter surface. Hence, the above described blood processing filter is easily adaptable to small centrifuge cups also, while retaining the filtration cross-sectional area thereof.

Further, the above described blood processing filter may have an approximately rectangular shape, and may be curved so as to have one pair of sides of an identical curved shape and another pair of sides that form parallel straight lines.

The present invention further provides a blood circuit system such as defined in claim 4.

According to the above described blood circuit system, the blood processing filter, the reservoir bag, and the recovery bag are stored together in a centrifuge cup when centrifuging blood that is in the reservoir bag. Generally, many centrifuge cups of this kind have a round shape such as a circular shape in a planar view or an elliptical shape in a planar view, and many of such centrifuge cups have a round inner surface such as a cylindrical surface or an elliptic cylindrical surface. Since the blood processing filter of the above described blood circuit system has a curved shape, the blood processing filter can be smoothly inserted into a centrifuge cup by aligning the curved shape of the blood processing filter with the curved shape of an inner wall surface of the centrifuge cup. Hence, an operation to store the blood processing filter in the centrifuge cup is performed with ease. Further, since the blood processing filter has a curved shape, the width thereof can be reduced while retaining the area of the filter surface. Hence, the above described blood circuit system easily adapts to a small centrifuge cup also, while retaining the filtration cross-sectional area of the blood processing filter.

The present invention also provides a method for centrifugation such as defined in claim 5.

According to the above described method for centrifugation, the blood processing filter, the reservoir bag, and the recovery bag are stored together in a centrifuge cup when centrifuging blood in the reservoir bag. Generally, many centrifuge cups of this kind have a round shape such as a circular shape in a planar view or an elliptical shape in a planar view, and many of such centrifuge cups have a round inner surface such as a cylindrical surface or an elliptic cylindrical surface. According to the above described method for centrifugation, the blood processing filter is arranged inside the centrifuge cup by aligning a curved face of the blood processing filter with the curved shape of an inner wall surface of the centrifuge cup. Hence, an operation to store the blood processing filter in the centrifuge cup is performed with ease. Further, since the blood processing filter has a curved shape, the width thereof can be reduced while retaining the area of the filter surface. Hence, the above described method for centrifugation easily adapts to a small centrifuge cup also, while retaining the filtration cross-sectional area of the blood processing filter.

### Advantageous Effects of Invention

According to the present invention, a blood processing filter, a blood circuit system, and a method for centrifugation can be provided that facilitate an operation to store the blood processing filter or blood circuit system in a centrifuge cup, and that easily adapt to a small centrifuge cup also, while retaining the filtration cross-sectional area.

### Brief Description of Drawings

Fig. 1 is a front view that illustrates a preferred embodiment of a blood processing filter according to the present invention;
Fig. 2 is a cross-sectional view along a line II-II of the blood processing filter shown in Fig. 1;
Fig. 3 is a cross-sectional view along a line III-III of the blood processing filter shown in Fig. 1;
Fig. 4 is a schematic diagram that illustrates a blood circuit system that includes the filter shown in Fig. 1;
Fig. 5 is a perspective view that illustrates one example of a centrifugal machine and a centrifuge cup;
Fig. 6 is a transverse cross-sectional view that illustrates a state in which a blood circuit system is stored in a centrifuge cup;
Fig. 7 is a view that illustrates a state in which blood in a reservoir bag has been separated into a plurality of components; and
Fig. 8 is a view that illustrates dimensions A, B, and C and the like that represent a curved shape of a blood processing filter.

### Description of Embodiments

### [Blood Processing Filter]

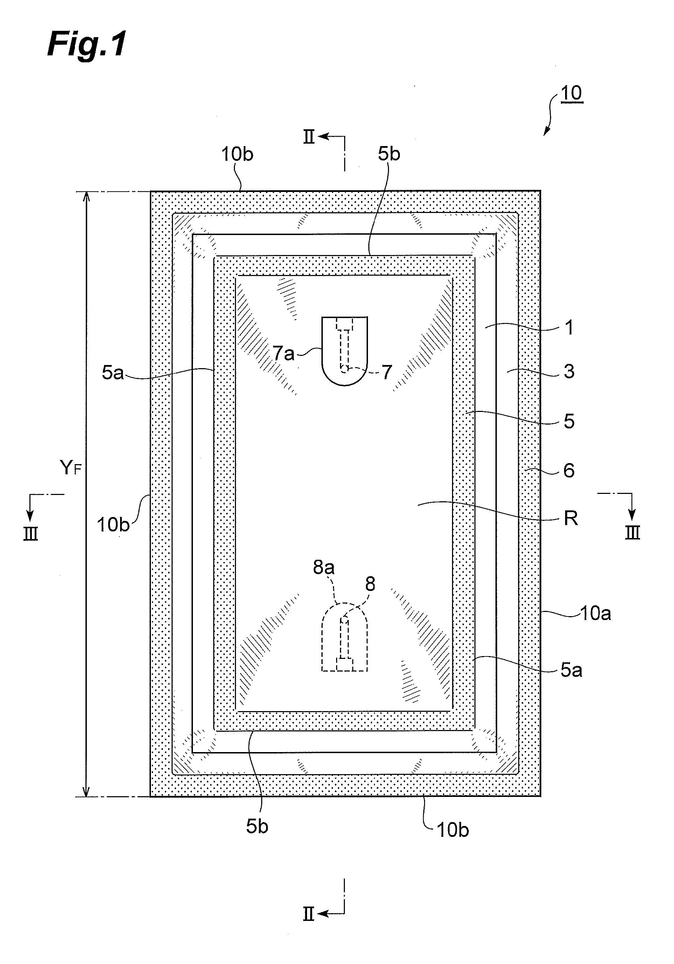

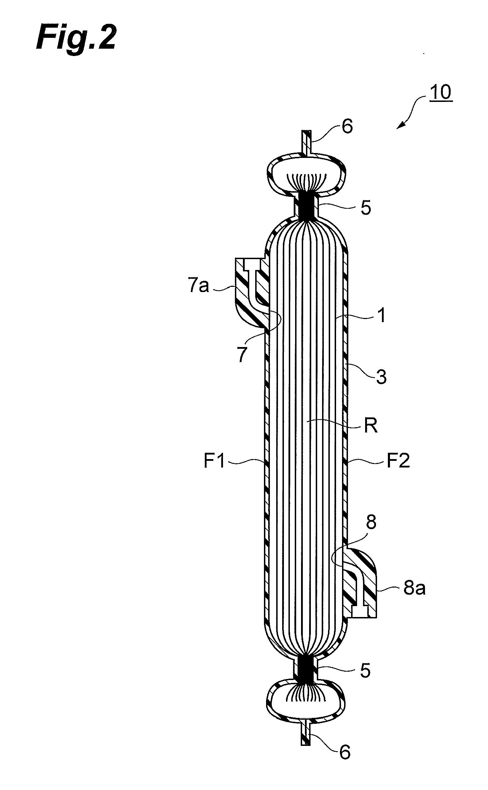

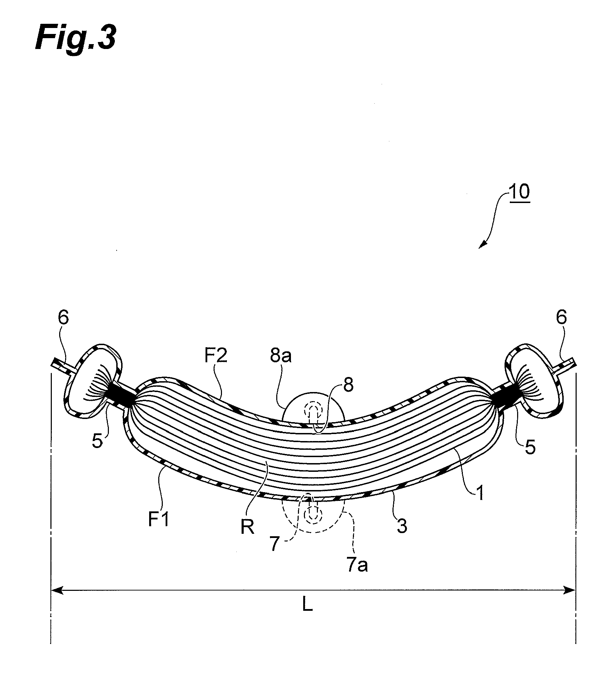

A blood processing filter 10 of the present embodiment that is shown in Fig. 1 to Fig. 3 is used for removing microaggregates and leukocytes which may cause side effects from blood components obtained by centrifugation. The filter 10 has a rectangular, flat shape that curves as viewed from a predetermined one direction (for example, in this case, as viewed from the upward direction of the page surface of Fig. 1) that is orthogonal to the thickness direction thereof. That is, the shape of the filter 10 in a front view is a rectangle as shown in Fig. 1, and as will be understood from Fig. 3, the filter 10, for example, curves along an arc as viewed at a cross section along a line III-III in Fig. 1. For example, an outer curved face F1 and an inner curved face F2 of the filter 10 form a cylindrical face.

The filter 10 has a pair of long sides 10a that form parallel straight lines, and a pair of short sides 10b that curve in the same shape as each other. The dimensions of the long sides 10a and the dimensions of the short sides 10b that are measured along a curve, of the filter 10 are appropriately set in a manner that takes into consideration a filtration cross-sectional area required for the filter 10 are the like.

As shown in the drawings, the filter 10 includes a sheet-like filter media 1, a flexible container 3 that houses the filter media 1, an inside joining portion 5 that defines a blood processing region R, and an outside joining portion 6.

The container 3 is constituted by a flexible sheet. An inlet 7 is provided on the curved face F1 side in the outward direction and an outlet 8 is provided on the curved face F2 side in the inward direction of the container 3. Ports 7a and 8a that are used for connecting to a tube are provided in the inlet 7 and the outlet 8, respectively. When causing an erythrocyte fraction to flow downward by gravitational force, the filter 10 is arranged in the vertical direction such that the inlet 7 is at an upper position and the outlet 8 is at a lower position so that blood that flows in from the inlet 7 flows vertically downward and reaches the outlet 8.

The inside joining portion 5 is formed in a belt-like, rectangular ring shape around the entire circumference that is adjacent to a peripheral portion of the filter media 1, and integrally joins the filter media 1 and the container 3. The inside joining portion 5 is a portion that is modified to have a property that does not allow blood to penetrate therethrough as a result of the filter media 1 and the container 3 being welded and hardened. Hence, blood that is introduced into the filter 10 from the inlet 7 is discharged from the outlet 8 without spreading to outside of the inside joining portion 5. More specifically, a space that is surrounded by the inside joining portion 5 is the blood processing region R for processing blood, and the inside joining portion 5 has a function that seals the blood processing region R. Furthermore, the filter media 1 divides the blood processing region R into a space on the inlet 7 side and a space on the outlet 8 side. Hence, after blood from the inlet 7 passes through the filter media 1 inside the blood processing region R, the blood is discharged from the outlet 8.

The inside joining portion 5 is hardened and has a certain level of rigidity. In contrast, the filter media 1 and the container 3 are constituted by a flexible material and are capable of deforming relatively easily. Accordingly, the overall three-dimensional shape of the filter 10 largely depends on the three-dimensional shape of the inside joining portion 5, and is retained by means of the rigidity of the inside joining portion 5.

In the filter 10, the inside joining portion 5 is formed in a curved shape. More specifically, the long sides 5a and 5a of the inside joining portion 5 that forms a rectangle in a front view form parallel straight lines. As viewed from a direction that is parallel to the long sides 5a and 5a, the short sides 5b and 5b curve in the same shape so as to form, for example, an arc. Because the inside joining portion 5 has the above described three-dimensional shape, the overall three-dimensional shape of the filter 10 is retained in the aforementioned curved shape by the rigidity of the inside joining portion 5.

The outside joining portion 6 seals the container 3 in the thickness direction of the filter 10 in order to protect the filter media 1 more reliably from the external environment. The outside joining portion 6 is provided so as to form the outer circumference of the rectangular container 3.

The filter 10 is manufactured by the following method. First, a material for a sheet-like filter media material is prepared that is capable of adsorbing aggregates or leukocytes included in blood. The aforementioned material is cut into a predetermined size to obtain the filter media 1. Cutting of the filter media material can be carried out using a knife, an ultrasonic cutter, or a laser cutter.

A flexible material is preferable as the filter media material, and examples thereof include a fiber structure such as a nonwoven fabric manufactured by a melt-blow method, a porous material (a sponge-like structure) having consecutive pores, and a porous membrane. A material composed of a fiber structure, a porous material, or a porous membrane, or a material that employs any of these materials as a base material and whose surface is chemically or physically modified may also be used as the filter media material. A single layer of a fiber structure, a porous material, or a porous membrane may be used as the filter media material, or a plurality of layers in which these materials are combined may be used as the filter media material.

Examples of the raw material of the above described fiber structure include polyester, polypropylene, polyamide, polyacrylonitrile, polytrifluoroethylene, polymethyl methacrylate, and polystyrene. When using a fiber structure as the filter media 1 or as a base material thereof, the fiber structure may be composed of fibers having an almost uniform diameter, or may be a material in which a plurality of types of fibers that differ in fiber diameter are commingled, as disclosed in International Publication No. WO 97/232266. In order to reduce the number of leukocytes in blood after filtration to 5×10⁶/unit, the average diameter of fibers forming the filter media 1 is preferably 3.0 µm or less, and more preferably is between 0.9 and 2.5 µm.

Examples of the raw material of the aforementioned porous material or porous membrane include polyacrylonitrile, polysulfone, cellulose acetate, polyvinyl formal, polyester, polyacrylate, polymethacrylate, and polyurethane. In order to reduce the number of leukocytes in blood after filtration to 5×10⁶/unit, the average pore diameter of the porous material or the porous membrane is preferably 2 µm or more and less than 10 µm.

Next, a resin sheet or film having flexibility for forming the container 3 is prepared. Examples of the raw material of the container 3 include: soft polyvinyl chloride; polyurethane; ethylene-vinyl acetate copolymer; polyolefin such as polyethylene and polypropylene; hydrogenated styrene-butadiene-styrene copolymer; a thermoplastic elastomer such as styrene-isoprene-styrene copolymer or the hydrogenated product thereof; and mixtures of the thermoplastic elastomer and a softening agent such as polyolefin, ethylene-ethyl acrylate, or the like. Of these, soft polyvinyl chloride, polyurethane, polyolefin, and thermoplastic elastomers containing these as a major component are particularly preferable as the raw material of the container 3, because they offer excellent permeability with respect to high-pressure steam or electron beams for sterilization, and also have toughness to withstand a load at a time of centrifugation.

By formation of the inside joining portion 5, the filter media 1 and the container 3 are integrally joined and the blood processing region R of the filter 10 is defined. The inside joining portion 5 and the outside joining portion 6 can be formed, for example, by high frequency welding.

The method of forming the inside joining portion 5 will now be described more specifically. A tabular intermediate product is prepared in which the filter media 1 is inserted between the two resin sheets forming the container 3. The resin sheets and the filter media 1 are inserted in a rectangular ring-shaped mold and subjected to high-frequency heating. At a rectangular ring-shaped heated portion that corresponds to the mold, the filter media 1 and the container 3 are integrally welded, and the hard inside joining portion 5 that has a rectangular ring shape is formed. In this process, by using a mold that has a curved shape, the inside joining portion 5 that has a curved shape corresponding to the shape of the mold can be formed while curving the aforementioned intermediate product.

The inlet port 7a and the outlet port 8a are respectively provided at predetermined positions of the container 3 by, for example, welding. It is sufficient that the inlet port 7a and the outlet port 8a allow the blood processing region R and the outside to communicate through the inlet 7 and the outlet 8. The filter 10 is manufactured by performing the above described process.

### [Blood Circuit System]

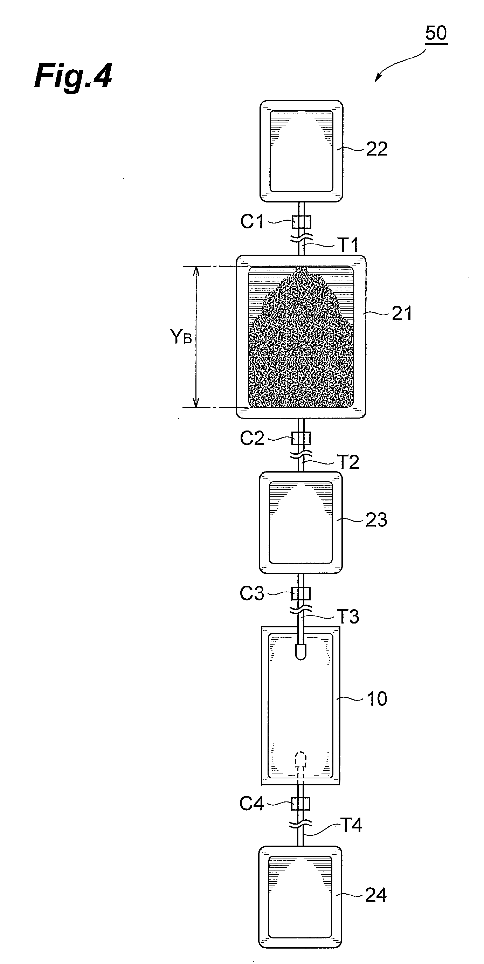

As shown in Fig. 4, a blood circuit system 50 of the present embodiment includes a reservoir bag 21 for holding blood. The blood circuit system 50 also includes a bag 22 for recovering a plasma fraction that communicates with the upper side of the reservoir bag 21 through a tube T1, and a bag 23 for recovering an erythrocyte fraction that communicates with the lower side of the reservoir bag 21 through a tube T2.

The blood circuit system 50 further includes the aforementioned filter 10 and a bag 24 for recovering an erythrocyte fraction. The inlet 7 of the filter 10 communicates with the bag 23 through a tube T3. The outlet 8 of the filter 10 communicates with the upper side of the bag 24 through a tube T4.

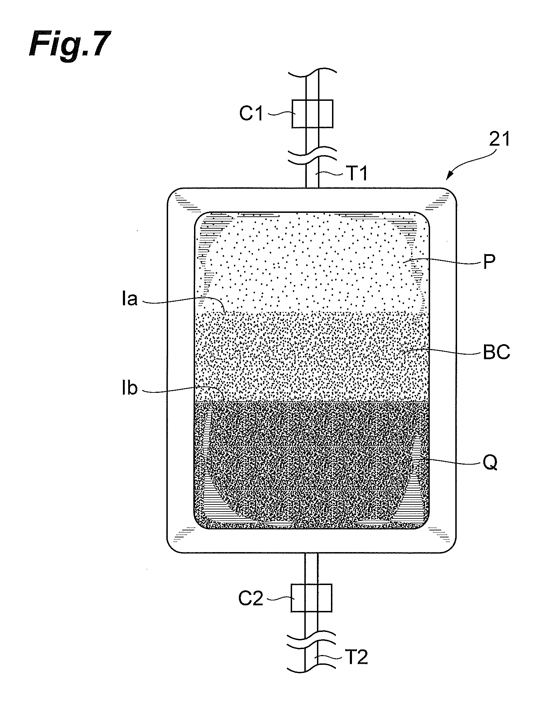

Clamps C1, C2, C3 and C4 are disposed partway along the tubes T1, T2, T3 and T4, respectively. In this connection, the bag 23 is used according to necessity, and is used for temporarily accumulating an erythrocyte fraction Q (see Fig. 7) inside the reservoir bag 21 prior to filtration by the filter 10.

Note that a configuration may also be adopted in which a blood processing filter is also disposed partway along the tube T1 to enable filtration of a plasma fraction P when transferring the plasma fraction P to the bag 22 through the tube T1.

### [Centrifugation Method]

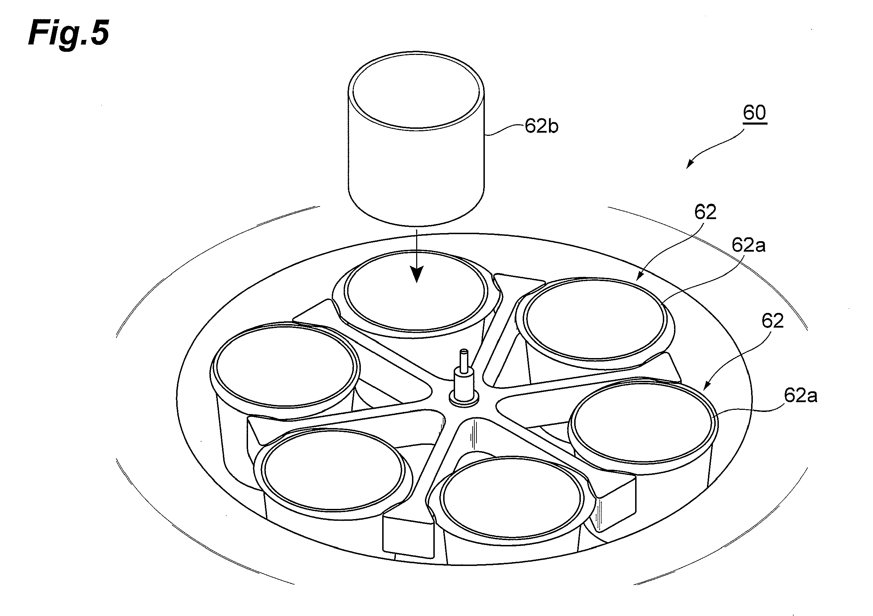

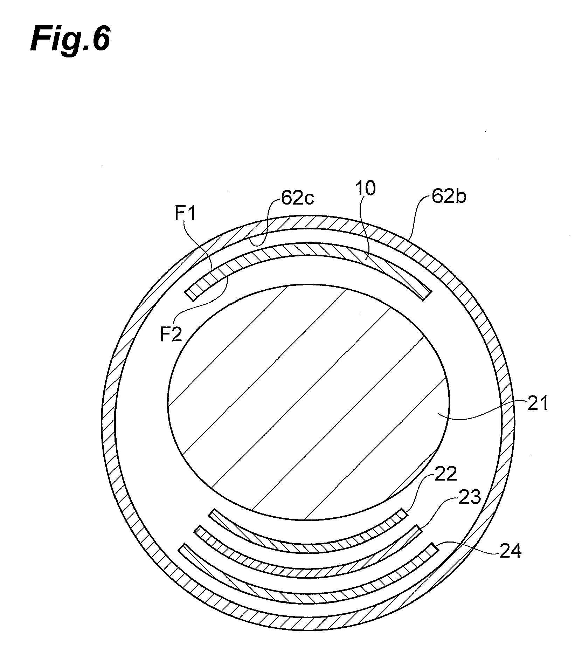

Centrifugation of the blood that has been recovered in the reservoir bag 21 is performed to separate and recover the plasma fraction P and the erythrocyte fraction Q from the blood inside the reservoir bag 21. In this case, the blood circuit system 50 is set in a bag holding portion 62 of a centrifugal machine 60 shown in Fig. 5 and centrifugation is performed. The bag holding portion 62 of the centrifugal machine 60 is constituted by an outer container 62a and a centrifuge cup 62b that is removably stored in the outer container 62a. A common type of centrifuge cup that has a round shape such as a circular shape in a planar view or an elliptical shape in a planar view can be used as the centrifuge cup 62b. In this case, as shown in Fig. 6, it is assumed that the centrifuge cup 62b has a circular shape in a planar view.

The centrifuge cup 62b has a size with a diameter of X_{C} and a depth of Y_{C}. It is desirable that a ratio (Y_{F}/Y_{C}) between a length Y_{F} in the longitudinal direction of the filter 10 and the depth Y_{C} of the centrifuge cup 62b is between 0.5 and 1.0, and a ratio between 0.7 and 0.9 is preferable. If the ratio Y_{F}/Y_{C} is less than 0.5, in some cases the dimensions of the filter 10 may be too small. In contrast, if the ratio Y_{F}/Y_{C} exceeds 1.0, there is a possibility that a portion of the filter 10 will protrude from the centrifuge cup 62b and interfere with the centrifugal machine, which may result in damage to the filter 10.

(Storing Step): As shown in Fig. 6, the entire blood circuit system 50 is stored inside the centrifuge cup 62b in a state in which the filter 10, the reservoir bag 21, and the bags 22, 23 and 24 are overlapping. In this case, the filter 10 is arranged so that the outer curved face F1 thereof is aligned with the curved shape of the inner wall surface 62c of the centrifuge cup 62b. It is preferable that the curved face F1 is a shape that curves completely along the inner wall surface 62c because such a shape facilitates insertion of the filter 10 into the centrifuge cup 62b and also allows the filter 10 to be stably mounted inside the centrifuge cup 62b.

The reservoir bag 21 is arranged on the inner curved face F2 side of the filter 10. Since the reservoir bag 21 that contains blood is roundish, it is easy to stably arrange the reservoir bag 21 along the curved face F2. Further, since the empty bags 22, 23 and 24 are relatively thin and also change shape easily, the bags 22, 23 and 24 are arbitrarily disposed in any of the remaining gaps. Next, the centrifuge cup 62b that accommodates the blood circuit system 50 in the above described manner is mounted inside the outer container 62a.
(Centrifugation Step): Subsequently, by starting the centrifugal machine 60 and rotating the centrifuge cup 62b for a predetermined time period, the blood in the reservoir bag 21 is centrifuged inside the reservoir bag 21. As a result, as shown in Fig. 7, the blood in the reservoir bag 21 is separated into the plasma fraction P, a buffy coat BC including leukocytes, and the erythrocyte fraction Q. Thereafter, the blood circuit system 50 is drawn out from the centrifuge cup 62b in a slow manner so as not to generate fluctuations at interfaces Ia and Ib due to deformation or wobbling of the reservoir bag 21.
(Component Recovery Step): Next, the erythrocyte fraction Q in the reservoir bag 21 is transferred to the bag 23 through the tube T2. Thereafter, the erythrocyte fraction Q in the bag 23 is transferred to the filter 10 through the tube T3, and leukocytes are removed therefrom by the filter media 1 of the filter 10. The erythrocyte fraction Q that has passed through and been filtered by the filter 10 is recovered in the recovery bag 24 through the tube T4. On the other hand, the plasma fraction P in the reservoir bag 21 is transferred to the bag 22 through the tube T1 and recovered.

Next, operational advantages of the above described filter 10, blood circuit system 50, and centrifugation method are described.

Because a blood processing filter of this kind has a hard inside joining portion, it is difficult to deform the blood processing filter when storing the blood processing filter in a centrifuge cup. Among the components constituting a blood circuit system, the blood processing filter is a relatively large component that is difficult to deform and consequently is a factor that makes the storing step complicated. However, since the filter 10 is retained in a curved shape as described above, the filter 10 can be smoothly inserted inside the centrifuge cup 62b by aligning the outer curved face F1 with the curved shape of the inner wall surface 62c of the centrifuge cup 62b. Hence, in comparison to a tabular blood processing filter that is not curved, an operation to store the filter 10 in the centrifuge cup 62b is easily carried out, and the step of storing the blood circuit system 50 in the centrifuge cup 62b is performed with ease.

Further, since the filter 10 is difficult to deform, it is necessary for the diameter of a centrifuge cup to which the blood circuit system 50 can be applied to be larger than the width of the filter 10. In this case, the term "width of the filter 10" refers to, as shown in Fig. 3, a linear distance L between two ends of the filter 10 in a planar view. In this connection, although it is also conceivable to forcedly deform the filter 10 and push the filter 10 into a centrifuge cup having a diameter that is smaller than the original width of the filter 10, this is not preferable because there is a risk that the inside joining portion 5 will break during centrifugation and blood will leak.

Since the filter 10 has a curved shape, the width thereof can be reduced while retaining the area of the filter surface. Hence, the filter 10 easily adapts to a small centrifuge cup also, while retaining the filtration cross-sectional area thereof. Because the filtration cross-sectional area of the filter 10 is retained, the time required for filtration of the erythrocyte fraction Q in the blood circuit system 50 is reduced.

Since use of the filter 10 also enables a reduction in the size of a centrifuge cup, it is possible to increase the number of centrifuge cups that are arranged in a centrifugal machine, and thus the efficiency of centrifugation work can be improved.

Further, as shown in Fig. 6, by adopting an arrangement in which the reservoir bag 21 is disposed in alignment with the curved shape of the filter 10, after centrifugation, by taking the filter 10 and the reservoir bag 21 out together from the centrifuge cup 62b, the reservoir bag 21 is protected by the filter 10 that is difficult to deform, and thus deformation or wobbling of the reservoir bag 21 can be suppressed when taking out the reservoir bag 21. Thus, the generation of fluctuations at the interfaces Ia and Ib due to deformation or wobbling of the reservoir bag 21 can be suppressed, and as a result an erythrocyte fraction and a plasma fraction can be recovered with a sufficiently high yield after centrifugation.

Note that the present invention is not limited to the above described embodiment. For example, according to the filter 10 of the embodiment, the long sides 10a form parallel straight lines and the short sides 10b are curved. However, according to the present invention a configuration may also be adopted in which the short sides of the filter form parallel straight lines and the long sides are curved. Further, although the inlet 7 is provided in the outer curved face F1 and the outlet 8 is provided in the inner curved face F2 in the filter 10, according to the present invention the outlet may be provided in the outer curved face and the inlet may be provided in the inner curved face. Furthermore, although according to the embodiment the filter 10 forms a curved shape along a cylindrical surface, according to the present invention, for example, the filter may form a curved shape along a different kind of cylindrical surface (for example, an elliptic cylindrical surface) or along a conical surface.

### (Examples)

The present inventors prepared curved-shaped blood processing filters for test use, and prepared respective blood circuit systems 50 (Fig. 4) in which each of the prepared blood processing filters for test use was applied to the filter 10. Subsequently, the present inventors carried out tests in which erythrocyte preparations were filtered using each of the prepared blood circuit systems 50 in the following manner. First, from a state in which blood was collected in the reservoir bag 21 of the blood circuit system 50, the blood circuit system 50 was inserted in a centrifuge cup and subjected to centrifugation using a centrifugal machine. Next, an erythrocyte fraction Q (erythrocyte preparation) that had separated inside the reservoir bag 21 was transferred to the bag 24 while being filtered with a blood processing filter for test use.

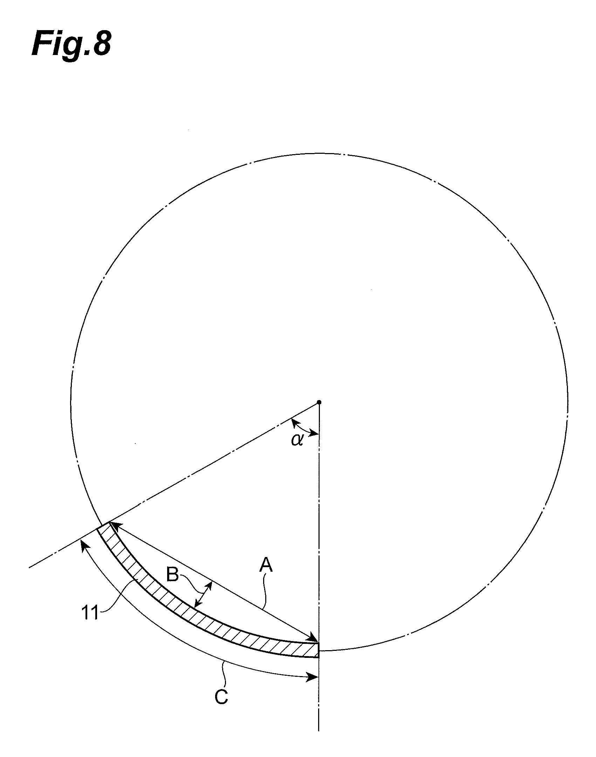

The present inventors prepared a total of four kinds of blood processing filters for test use which, as shown in Table 1, consisted of examples 1 and 2, a comparative example, and a reference example. Of these, the filters of examples 1 and 2 were curved so as to have a cylindrical surface, and as shown in Fig. 8, an effective filtration portion 11 of each filter was also curved so as to have a cylindrical surface. Note that the term "effective filtration portion 11" refers to a portion of the filter media 1 inside the blood processing region R.

As shown in Fig. 8, the curved shape of the effective filtration portion 11 can be expressed by a chord length A, a maximum gap B that is formed between the chord and the effective filtration portion 11, and an arc length C. The values of A, B, and C in each filter are as shown in Table 1. Note that the filters of the comparative example and the reference example are a tabular shape without a curve, and hence the length B thereof is zero. The longitudinal lengths of the effective filtration portions 11 of the respective filters were all equal.

The filter media of each filter included first to third filter elements as described below. The filter media of each filter was produced by stacking four sheets of a first filter element, one sheet of a second filter element, 32 sheets of a third filter element, one sheet of the second filter element, and four sheets of the first filter element in that order. The filter elements had the following specifications:
First filter element: fiber diameter 12 µm, mass per unit area 30g/m, thickness 0.2 mm
Second filter element: fiber diameter 1.6 µm, mass per unit area 66g/m, thickness 0.4 mm
Third filter element: fiber diameter 1.2 µm, mass per unit area 40g/m, thickness 0.2 mm

In this connection, the first filter elements function as a prefilter that removes contaminants and the like, and as a post-filter that secures a space on the outlet side. The second and third filter elements function as a main filter that removes leukocytes and the like.

The samples filtered by the respective filters were erythrocyte preparations that were stored for three days at 4°C. The filtration conditions consisted of filtration at room temperature with a filtering drop of 1.2 m, and the filter outlet tube length was 60 cm. For each filter, a time from initial flow of the sample to the filter from the reservoir bag until the sample disappeared from the reservoir bag, filter inlet tube, and a filtration surface on the inlet side of the filter was measured. The measured times are shown in Table 1 as filtration times.

In this connection, the long-axis width of a centrifuge cup of the centrifuge apparatus used for the tests was 65 mm. However, since the filter of the reference example had a length A of 72 mm, it was not possible to accommodate the filter of the reference example in a centrifuge cup and therefore centrifugation of the reference example could not be performed. With respect to the filter of the reference example, a filtration time in a case where an erythrocyte preparation was filtered under similar conditions as examples 1 and 2 and the comparative example is shown in Table 1 as a reference.

**[Table 1]**

| | Filter length × width | Length A | Length B | Length C | Filtration cross-sectional area | Filtration time |
|---|---|---|---|---|---|---|
| Example 1 | 74 mm × 61 mm | 57 mm | 9 mm | 61 mm | 45.2 cm² | 250 min |
| Example 2 | 74 mm × 75 mm | 57 mm | 21 mm | 75 mm | 55.5 cm² | 151 min |
| Comparative Example | 74 mm × 57 mm | 57 mm | 0 mm | 57 mm | 42.2 cm² | 317 min |
| Reference Example | 74 mm × 72 mm | 72 mm | 0 mm | 72 mm | 53.3 cm² | 188 min |

Since the length A was the same for each of examples 1 and 2 and the comparative example, the respective blood processing filters thereof could be stored in centrifuge cups having the same internal diameter. In contrast, the length C differed among the examples 1 and 2 and the comparative example, being longest in example 2 (75 mm), followed by example 1 (61 mm), and the comparative example (57 mm) in length order. Hence, the sizes of the respective filtration cross-sectional areas also differed, the filtration cross-sectional area being largest in example 2 (55.5 mm²), followed by example 1 (45.2 mm²), and the comparative example (42.2 mm²) in size order. Due to the differences in the filtration cross-sectional areas, example 2 (151 min) had the shortest filtration time, followed by example 1 (250 min), and the comparative example (317 min). Based on the above results, it was confirmed that even when the length A is the same, in comparison to the tabular filter of the comparative example, the filtration times were shorter for the filters of examples 1 and 2 that had a curved shape. Of these, it was confirmed that example 2 that had the longest length B (largest curvature) had the shortest filtration time.

Further, comparing example 2 and the reference example, it was found that the filtration cross-sectional areas were approximately equal and that the filtration times were also approximately equal. However, because the filter of the reference example had a tabular shape, the length A thereof was large and, as described above, the reference example could not be stored in a centrifuge cup having a long-axis width of 65 mm. Hence, it was confirmed that the range of application of a curved-shaped filter with respect to small centrifuge cups is broadened in comparison to a tabular filter having the same filtration cross-sectional area and for which a filtration time is also the same.

Thus, it was confirmed that by making a blood processing filter in a curved shape, the adaptation range thereof is broadened to smaller centrifuge cups while retaining the filtration cross-sectional area and maintaining the filtration time.

Note that a filter that has a curved shape such that an angle α shown in Fig. 8 is 180° or more is not preferable because there is a concern that the inside joining portion 5 will crack when transporting the product. From the viewpoint of preventing cracking of the inside joining portion 5, it is preferable that a value of length A/length C is 0.6 or more.

### Industrial Applicability

According to the present invention, by making a blood processing filter a curved shape, an operation to store a blood circuit system in a centrifuge cup is facilitated, and it is possible to easily adapt to small centrifuge cups also, while retaining the filtration cross-sectional area of the blood processing filter.

### Reference Signs List

- 1: filter media
- 3: flexible container
- 5: inside joining portion (seal part)
- 7: inlet
- 8: outlet
- 10: blood processing filter
- 10a: long sides
- 10b: short sides
- 21: reservoir bag
- 62b: centrifuge cup
- 62c: inner wall surface
- T1-T4: tube

## Claims

1. A blood processing filter (10) comprising a flexible container (3) having an inlet (7) and an outlet (8) for blood, and a sheet-like filter media (1) arranged so as to divide inside of the flexible container (3) into one side and another side; wherein:
an annular seal part (5) is formed by integrally sealing the complete periphery of the filter media (1) and the flexible container (3) at near the very edge of the filter media (1);
the blood processing filter (10) is retained in a curved shape that curves as viewed from a predetermined one direction that is orthogonal to the thickness direction by rigidity of the seal part (5),
the surface provided with the inlet (7) and the other surface provided with the outlet (8) are curved in the same one direction as viewed from the predetermined one direction, and
the inlet (7) is provided on the curved face (F1) side in the outward direction and the outlet (8) is provided on the curved face (F2) in the inward direction, wherein the curvature is in the same direction as viewed from the predetermined one direction.

2. The blood processing filter (10) according to claim 1, wherein the filter media (1) is curved as viewed from the predetermined one direction.

3. The blood processing filter (10) according to claim 1 or 2, wherein the blood processing filter (10) has an approximately rectangular shape, and is curved so as to have one pair of sides of a curved shape being curved in the same one direction as viewed from the predetermined one direction and another pair of sides that form parallel straight lines extending in the predetermined one direction.

4. A blood circuit system (50) that comprises a reservoir bag (21) that contains blood, a blood processing filter (10) according to any one of claims 1 to 3 that is connected to the reservoir bag (21) and is used for filtering a blood component obtained by centrifuging blood in the reservoir bag (21), and a recovery bag (24) that is connected to the blood processing filter (10) and is used to contain the blood component;
wherein the blood processing filter (10) comprises: a flexible container (3) having an inlet (7) that communicates with the reservoir bag (21) through a tube (T2), and an outlet (8) that communicates with the recovery bag (24) through a tube (T4).

5. A method for centrifugation comprising:
a storing step of storing, in a centrifuge cup (62b) of a centrifugal machine, a blood circuit system comprising a reservoir bag (21) that contains blood, a blood processing filter (10) according to any one of claims 1 to 3 that is connected to the reservoir bag (21) and that is used for filtering a blood component obtained by centrifuging blood in the reservoir bag (21), and a recovery bag (24) that is connected to the blood processing filter (10) and that is used to contain the blood component; and
a centrifugation step of centrifuging the blood in the reservoir bag (21) by rotating the centrifuge cup (62b) in which the blood circuit system is stored using the centrifugal machine (60); wherein:
the blood processing filter (10) comprises: a flexible container (3) having an inlet (7) that communicates with the reservoir bag (21) through a tube (T2), and an outlet (8) that communicates with the recovery bag (24) through a tube (T4); and
in the storing step, the blood processing filter (10) is arranged inside the centrifuge cup (62b) by aligning a curved face of the blood processing filter, the curved face being one of the surface or the other surface, with a curved shape of an inner wall surface of the centrifuge cup (62b).

## Patentansprüche

1. Blutverarbeitungsfilter (10), umfassend einen flexiblen Behälter (3) mit einem Einlass (7) und einem Auslass (8) für Blut und ein flächiges Filtermedium (1), das so angeordnet ist, dass es das Innere des flexiblen Behälters (3) in eine Seite und eine andere Seite unterteilt; wobei
ein ringförmiges Dichtungsteil (5) gebildet wird, indem man den gesamten Rand des Filtermediums (1) und des flexiblen Behälters (3) in der Nähe des äußersten Randes des Filtermediums (1) integral versiegelt;
der Blutverarbeitungsfilter (10) durch die Steifigkeit des Dichtungsteils (5) in einer gekrümmten Form gehalten wird, die aus der Sicht einer vorbestimmten einen Richtung, welche senkrecht zur Dickenrichtung verläuft, gekrümmt ist;
die mit dem Einlass (7) versehene Fläche und die andere Fläche, die mit dem Auslass (8) versehen ist, aus der Sicht der vorbestimmten einen Richtung in derselben einen Richtung gekrümmt sind; und
sich der Einlass (7) auf der Seite mit der gekrümmten Fläche (F1) in Richtung nach außen befindet und sich der Auslass (8) auf der gekrümmten Fläche (F2) in Richtung nach innen befindet, wobei die Krümmung aus der Sicht der vorbestimmten einen Richtung in derselben Richtung verläuft.

2. Blutverarbeitungsfilter (10) gemäß Anspruch 1, wobei das Filtermedium (1) aus der Sicht der vorbestimmten einen Richtung gekrümmt ist.

3. Blutverarbeitungsfilter (10) gemäß Anspruch 1 oder 2, wobei der Blutverarbeitungsfilter (10) eine ungefähr rechteckige Form aufweist und so gekrümmt ist, dass er ein Seitenpaar mit einer gekrümmten Form, die aus der Sicht der vorbestimmten einen Richtung in derselben Richtung gekrümmt sind, und ein anderes Seitenpaar, die parallele Geraden bilden, welche sich in der vorbestimmten einen Richtung erstrecken, aufweist.

4. Blutkreislaufsystem (50), umfassend einen Vorratsbeutel (21), der Blut enthält, einen Blutverarbeitungsfilter (10) gemäß einem der Ansprüche 1 bis 3, der mit dem Vorratsbeutel (21) verbunden ist und zum Abfiltrieren einer Blutkomponente, die durch Zentrifugieren von Blut in dem Vorratsbeutel (21) erhalten wird, verwendet wird, und einen Auffangbeutel (24), der mit dem Blutverarbeitungsfilter (10) verbunden ist und verwendet wird, um die Blutkomponente aufzufangen;
wobei der Blutverarbeitungsfilter (10) einen flexiblen Behälter (3) mit einem Einlass (7), der über einen Schlauch (T2) mit dem Vorratsbeutel (21) verbunden ist, und einem Auslass (8), der über einen Schlauch (T4) mit dem Auffangbeutel (24) verbunden ist, umfasst.

5. Zentrifugationsverfahren, umfassend:
einen Aufbewahrungsschritt zum Aufbewahren eines Blutkreislaufsystems, das einen Vorratsbeutel (21), der Blut enthält, einen Blutverarbeitungsfilter (10) gemäß einem der Ansprüche 1 bis 3, der mit dem Vorratsbeutel (21) verbunden ist und zum Abfiltrieren einer Blutkomponente, die durch Zentrifugieren von Blut in dem Vorratsbeutel (21) erhalten wird, verwendet wird, und einen Auffangbeutel (24), der mit dem Blutverarbeitungsfilter (10) verbunden ist und verwendet wird, um die Blutkomponente aufzufangen, umfasst, in einem Zentrifugenbecher (62b) einer Zentrifugenmaschine; und
einen Zentrifugationsschritt zum Zentrifugieren des Bluts in dem Vorratsbeutel (21) durch Rotierenlassen des Zentrifugenbechers (62b), in dem das Blutkreislaufsystem aufbewahrt wird, mit Hilfe der Zentrifugenmaschine (60); wobei
der Blutverarbeitungsfilter (10) einen flexiblen Behälter (3) mit einem Einlass (7), der über einen Schlauch (T2) mit dem Vorratsbeutel (21) verbunden ist, und einem Auslass (8), der über einen Schlauch (T4) mit dem Auffangbeutel (24) verbunden ist, umfasst; und
in dem Aufbewahrungsschritt der Blutverarbeitungsfilter (10) dadurch innerhalb des Zentrifugenbechers (62b) angeordnet wird, dass man eine gekrümmte Fläche des Blutverarbeitungsfilters, wobei die gekrümmte Fläche die eine Fläche oder die andere Fläche ist, an einer gekrümmten Fläche einer Innenwandfläche des Zentrifugenbechers (62b) ausrichtet.

## Revendications

1. Filtre de traitement de sang (10) comprenant un récipient souple (3) ayant une entrée (7) et une sortie (8) pour le sang, et une couche filtrante de type feuille (1) agencée afin de diviser l'intérieur du récipient souple (3) en un côté et en un autre côté ; dans lequel :
une partie de joint d'étanchéité annulaire (5) est formée en scellant de manière solidaire toute la périphérie de la couche filtrante (1) et le récipient souple (3) à côté du bord de la couche filtrante (1) ;
le filtre de traitement de sang (10) est retenu dans une forme incurvée qui s'incurve lorsqu'elle est observée à partir d'une direction prédéterminée qui est orthogonale à la direction d'épaisseur, par la rigidité de la partie de joint d'étanchéité (5),
la surface prévue avec l'entrée (7) et l'autre surface prévue avec la sortie (8) sont incurvées dans la même direction, lorsqu'elle est observée dans la une direction prédéterminée, et
l'entrée (7) est prévue du côté de la face incurvée (F1) dans la direction vers l'extérieur et la sortie (8) est prévue sur la face incurvée (F2) dans la direction vers l'intérieur, dans lequel la courbure est dans la même direction, lorsqu'elle est observée à partir de la une direction prédéterminée.

2. Filtre de traitement de sang (10) selon la revendication 1, dans lequel la couche filtrante (1) est incurvée lorsqu'elle est observée à partir de la une direction prédéterminée.

3. Filtre de traitement de sang (10) selon la revendication 1 ou 2, dans lequel le filtre de traitement de sang (10) a une forme approximativement rectangulaire, et est incurvé afin d'avoir une paire de côtés d'une forme incurvée qui sont incurvés dans la même direction, lorsqu'ils sont observés à partir de la une direction prédéterminée et l'autre paire de côtés qui forment des lignes droites parallèles s'étendant dans la une direction prédéterminée.

4. Système de circuit de sang (50) qui comprend un sachet formant réservoir (21) qui contient du sang, un filtre de traitement de sang (10) selon l'une quelconque des revendications 1 à 3, qui est raccordé au sachet formant réservoir (21) et est utilisé pour filtrer un composant sanguin obtenu en centrifugeant le sang dans le sachet formant réservoir (21), et un sachet de récupération (24) qui est raccordé au filtre de traitement de sang (10) et est utilisé pour contenir le composant sanguin ;
dans lequel le filtre de traitement de sang (10) comprend : un récipient souple (3) ayant une entrée (7) qui communique avec le sachet formant réservoir (21) à travers un tube (T2), et une sortie (8) qui communique avec le sachet de récupération (24) à travers un tube (T4).

5. Procédé de centrifugation comprenant :
une étape de stockage consistant à stocker, dans une coupelle à centrifuger (62b) d'une machine centrifuge, un système de circuit de sang comprenant un sachet formant réservoir (21) qui contient du sang, un filtre de traitement de sang (10) selon l'une quelconque des revendications 1 à 3 qui est raccordé au sachet formant réservoir (21) et qui est utilisé pour filtrer un composant sanguin obtenu en centrifugeant le sang dans le sachet formant réservoir (21), et un sachet de récupération (24) qui est raccordé au filtre de traitement de sang (10) et qui est utilisé pour contenir le composant sanguin ; et
une étape de centrifugation consistant à centrifuger le sang dans le sachet formant réservoir (21) en faisant tourner la coupelle à centrifuger (62b) dans laquelle le système de circuit de sang est stocké, en utilisant la machine centrifuge (60) ; dans lequel :
le filtre de traitement de sang (10) comprend : un récipient souple (3) ayant une entrée (7) qui communique avec le sachet formant réservoir (21) par le biais d'un tube (T2), et une sortie (8) qui communique avec le sachet de récupération (24) par le biais d'un tube (T4) ; et
à l'étape de stockage, le filtre de traitement de sang (10) est agencé à l'intérieur de la coupelle centrifuge (62b) en alignant une face incurvée du filtre de traitement de sang, la face incurvée étant l'une parmi la surface ou l'autre surface, avec une forme incurvée d'une surface de paroi interne de la coupelle à centrifuger (62b).
